# EUROPÄISCHE PATENTSCHRIFT

(11) **EP 0 982 287 B1**
(45) Veröffentlichungstag und Bekanntmachung des Hinweises auf die Patenterteilung: **29.10.2003**
(21) Anmeldenummer: 99116158.9
(22) Anmeldetag: 20.08.1999
(51) Int. Cl.: C07C 51/44, C07C 57/07

(54) **Verfahren zur kontinuierlichen Gewinnung von (Meth)acrylsäure**
Process for the continuous recovery of (meth)acrylic acid
Procédé pour la récupération en continu d'acide (méth)acrylique

(30) Priorität: 26.08.1998 DE 19838783
(43) Veröffentlichungstag der Anmeldung: 01.03.2000
(73) Patentinhaber: BASF AKTIENGESELLSCHAFT, 67056 Ludwigshafen (DE)
(72) Erfinder: Machhammer, Otto, Dr., 68163 Mannheim (DE); Haupt, Susanne, Dr., 63069 Offenbach (DE); Schliephake, Volker, Dr., 67105 Schifferstadt (DE); Schröder, Jürgen, Dr., 67071 Ludwigshafen (DE)
(74) Vertreter: Isenbruck, Günter, Dr.

(56) Entgegenhaltungen:
- EP-A- 0 722 926
- DE-A- 2 136 396
- DE-A- 2 834 140
- DE-A- 4 308 087
- DE-A- 19 746 690

## Beschreibung

Die Erfindung betrifft ein Verfahren zur kontinuierlichen Gewinnung von (Meth)acrylsäure durch Absorption von (Meth)acrylsäure aus den Reaktionsgasen einer katalytischen Gasphasenoxidation. Der Begriff (Meth)acrylsäure steht im folgenden für die Substanzen Acrylsäure und/oder Methacrylsäure.

(Meth)acrylsäure wird überwiegend durch katalytische Gasphasenoxidation geeigneter Ausgangsstoffe, insbesondere von Propen und/oder Acrolein im Falle der Acrylsäure bzw. von Isobuten und/oder Methacrolein im Falle der Methacrylsäure, hergestellt.

Zur Abtrennung der (Meth)acrylsäure aus den Reaktionsgasen der katalytischen Gasphasenoxidation sind eine Reihe von Möglichkeiten bekannt, darunter auch die Abtrennung durch Absorption in ein Lösungsmittel.

Aus DE-B 21 36 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DE-A 24 49 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten, insbesondere bei der destillativen Reinigung der (Meth)acrylsäure, der Anfall von Feststoffen in den Apparaten, der die Anlagenverfügbarkeit reduziert. Gemäß DE-A 43 08 087 kann im Fall der Acrylsäure dieser Feststoffanfall reduziert werden, indem man dem relativ unpolaren Lösungsmittelgemisch aus Diphenylether und Diphenyl ein polares Lösungsmittel wie Dimethylphthalat in einer Menge von 0,1 bis 25 Gew.-% zufügt; dadurch erhöht sich das Aufnahmevermögen des Lösungsmittelgemisches für die schmutzbildenden Stoffe. Mit steigender Polarität nimmt das Lösungsmittel jedoch zunehmende Mengen an Wasser mit auf; außerdem führt dies zu erhöhten Lösungsmittelverlusten über das Sauerwasser.

In Gegenwart von Lösungsmitteln bildet die Polyacrylsäure im Bereich höherer Temperaturen, wie sie bei der Gewinnung von (Meth)acrylsäure nach dem gattungsgemäßen Verfahren, insbesondere am untersten Sammelboden der Absorptionskolonne, im Abtriebs- und Sumpfteil der Destillationskolonne sowie in den Wärmetauschern auftreten, einen an der Oberfläche der Apparate fest haftenden Schmutz, der nur mit Laugen gelöst werden kann. Analysen haben gezeigt, daß der Schmutz aus einer Mischung aus ca. 10 bis 50 Gew.-% Poly(meth)acrylsäure, Rest Lösungsmittel, besteht.

Es wird bereits länger vermutet, daß die Polymerisationsneigung von (Meth)acrylsäure durch Leichtsieder gefördert wird.

Der Zusatz von Polymerisationsinhibitoren ist beispielsweise in Ullmanns Encyklopädie der techn. Chemie, 4. Aufl. Bd.7, S. 81, linke Spalte, beschrieben. Als Inhibitoren werden insbesondere Phenothiazin oder Hydrochinon in Mindestmengen von 500 ppm vorgeschlagen, sie haben jedoch den Nachteil, daß sie teuer sind, und zudem keine vollständige Inhibierung bewirken können.

Aus EP-A 0 722 926 ist es bekannt, aus dem Ablauf der Absorptionskolonne, worin das Reaktionsgemisch der katalytischen gasweisen Oxidation zu Acrylsäure in ein hochsiedendes Lösungsmittel absorbiert wurde, Leichtsieder durch Strippen mit Stickstoff zu entfernen. Beim Strippen wird ein Inertgasstrom (Stickstoff) im Gegenstrom zum beladenen Lösungsmittel geführt. Das Absorpt, vorliegend die Leichtsieder, wandert aus der Flüssigphase in die Gasphase, wobei sein Partialdruck in der Gasphase durch ständig zugeführtes Inertgas niedrig gehalten wird. Um diese Forderung zu erfüllen, muß bei der Desorption durch Strippen der Strippgasstrom stets wesentlich größer sein im Vergleich zum zu reinigenden Strom. Indem der Partialdruck des Absorpts, d.h. vorliegend der Leichtsieder, im Desorptionsverfahren durch Strippen somit definitionsgemäß niedrig gehalten wird, ist es bei diesem Verfahren prinzipiell nicht möglich, einen flüssigen Ablauf aus der Desorptionskolonne zu erhalten, der vollständig oder nahezu vollständig frei von Absorpt, d.h. von Leichtsiedem, ist.

Aufgabe der Erfindung ist es, die Polymerisationsneigung von (Meth)acrylsäure in einem Verfahren zur kontinuierlichen Gewinnung von (Meth)acrylsäure aus einem flüssigen Gemisch mit einem hochsiedenden Lösungsmittel sowie mit Leichtsiedern, Mittelsiedern und Schwersiedern zu verringern.

Die Lösung geht aus von einem Verfahren zur kontinuierlichen Gewinnung von (Meth)acrylsäure aus einem flüssigen Ausgangsgemisch mit einem Gehalt an (Meth)acrylsäure, einem hochsiedenden organischen Lösungsmittel sowie mit Leichsiedern, Mittelsiedern und Schwersiedem. Die Erfindung ist dann dadurch gekennzeichnet, daß
I das Gemisch destillativ in einer Abtriebskolonne in einen ersten Teilstrom (a), der neben (Meth)acrylsäure die Leichtsieder sowie jeweils einen Teil der Mittelsieder und Schwersieder enthält, und einen zweiten Teilstrom (b) aufgetrennt wird, der den überwiegenden Teil der (Meth)acrylsäure enthält und der vollständig oder nahezu vollständig frei von Leichtsiedern ist, indem dampfförmige, aus dem Sumpf der Abtriebskolonne aufsteigende (Meth)acrylsäure die Leichtsieder ohne Zuführung von Inertgas aus der Flüssigkeit strippt,
   und daß
II die (Meth)acrylsäure aus dem Teilstrom (b) destillativ in einer Auftriebskolonne gewonnen wird.

Es wurde gefunden, daß die die Polymerisation von (Meth)acrylsäure fördernde Wirkung von Leichtsiedern bei erhöhter Temperatur in überraschender Weise dadurch gemindert werden kann, daß die Leichtsieder im Beisein der Schwersieder abgetrennt werden.

Als hochsiedend werden vorliegend Lösungsmittel bezeichnet, deren Siedepunkt höher ist als der Siedepunkt des jeweils angestrebten Hauptprodukts (ca. 141°C für Acrylsäure bzw. ca. 161°C für Methacrylsäure, jeweils bei Normaldruck).

Ausgangsgemische für das vorliegende Verfahren sind die Reaktionsgase aus der katalytischen Gasphasenoxidation von C₃-Alkanen, -Alkenen, -Alkanolen und/oder -Alkanalen oder Vorstufen davon zu Acrylsäure bzw. von C₄-Alkanen, C₃-Alkanen, -Alkenen, -Alkanolen und/oder -Alkanalen oder Vorstufen davon zu (Meth)acrylsäure. Das Verfahren wird im folgenden für Acrylsäure beschrieben, es gilt jedoch analog auch für Methacrylsäure.

Besonders vorteilhaft ist die katalytische Gasphasenoxidation von Propen und/oder Acrolein zu Acrylsäure mit Luft oder molekularem Sauerstoff nach bekannten Verfahren, insbesondere wie sie in den oben genannten Druckschriften beschrieben sind. Vorzugsweise wird hierbei bei Temperaturen zwischen 200 und 450 °C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch katalytische Oxidehydrierung, wie in US-5 510 558 beschrieben oder durch homogene Oxidehydrierung, wie z. B. in CN-A-1 105 352 beschrieben; oder durch katalytische Dehydrierung, entsprechend zum Beispiel der EP-A-0 253 409. Bei Einsatz eines Propen-/Propan-Gemischs wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Kreisgas (siehe unten), Luftstickstoff, einen oder mehrere gesättigte C₁- bis C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist salzbadgekühlte Rohrbündelwärmetauscher verwendet, die mit dem Oxidationskatalysator gefüllt sind, da bei diesen die bei der Reaktion freiwerdende Wärme sehr gut durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Säuren und Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% der Summe aus Maleinsäure und Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 5 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Reaktionsgas, enthalten.

Aus dem Reaktionsgas der katalytischen Gasphasenoxidation werden die Acrylsäure und ein Teil der Nebenkomponenten durch Absorption in einem hochsiedenden Lösungsmittel abgetrennt. Vorzugsweise liegt der Siedepunkt des hochsiedenden Lösungsmittels wenigstens 20°C, insbesondere 50°C, stärker bevorzugt 70°C über dem Siedepunkt der Acrylsäure bzw. Methacrylsäure. Bevorzugte Lösungsmittel, wobei in vorliegender Anmeldung der Begriff Lösungsmittel auch Lösungsmittelgemische umfaßt, haben Siedepunkte (bei Normaldruck) von 180 bis 400°C, insbesondere von 220 bis 360°C. Geeignete Lösungsmittel sind hochsiedende, extrem hydrophobe Lösungsmittel, die keine nach außen wirkende polare Gruppe enthalten, wie aliphatische oder aromatische Kohlenwasserstoffe, z.B. Mittelölfraktionen aus der Paraffindestillation, oder Äther mit sperrigen Gruppen am O-Atom, oder Gemische davon, wobei diesen vorteilhafterweise ein polares Lösungsmittel, wie das in DE-A-43 08 087 offenbarte 1,2-Dimethylphthalat zugesetzt wird. Weiterhin eignen sich Ester der Benzoesäure und Phthalsäure mit geradkettigen, 1 bis 8 Kohlenstoffatome enthaltenden Alkanolen, wie Benzoesäuren-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkane, z.B. 4-Methyl-4'-benzyl-diphenylmethan und dessen Isomere 2-Methyl-2'-benzyl-diphenylmethan, 2-Methyl-4'-benzyl-diphenylmethan und 4-Methyl-2'-benzyl-diphenylmethan und Gemische solcher Isomerer.

Ein besonders bevorzugtes Lösungsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, bezogen auf 100 Gew.-% Diphenyl und Diphenylether, beispielsweise das im Handel erhältliche Diphyl®. Vorzugsweise enthält dieses Lösungsmittelgemisch weiterhin ein polares Lösungmittel wie Dimethylphthalat in einer Menge von 0,1 bis 25 Gew.-%, bezogen auf das gesamte Lösungsmittelgemisch. Dadurch reduziert sich die Verschmutzungsanfälligkeit der Anlagen.
Vorliegend bezeichnen die Begriffe Hoch- oder Schwersieder, Mittelsieder und Leichtsieder sowie entsprechend adjektivisch gebrauchte Begriffe Verbindungen, die einen höheren Siedepunkt als die Acrylsäure besitzen (Hochsieder) bzw. solche, die in etwa den gleichen Siedepunkt wie Acrylsäure besitzen (Mittelsieder) bzw. solche, die einen niedrigeren Siedepunkt als Acrylsäure besitzen (Leichtsieder).

Vorteilhafterweise wird das heiße Reaktionsgas durch Teilverdampfen des Lösungsmittels in einem Direktkondensator oder Quenchapparat, vor der Absorption abgekühlt. Hierfür eignen sich insbesondere Venturiwäscher, Blasensäulen oder Sprühkondensatoren. Dabei kondensieren die schwersiedenden Nebenkomponenten des Reaktionsgases in das nicht verdampfte Lösungsmittel. Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10 % des der Absorptionskolonne zugeführten Massenstroms, abgezogen und einer Lösungsmittelreinigung unterworfen. Hierbei wird das Lösungsmittel überdestilliert und zurück bleiben die schwersiedenden Nebenkomponenten, die - bei Bedarf weiter eingedickt - entsorgt, z.B. verbrannt, werden können. Diese Lösungsmitteldestillation dient der Vermeidung einer zu hohen Konzentration an Schwersiedern im Lösungsmittelstrom. Das überdestillierte Lösungsmittel wird vorzugsweise dem beladenen Lösungsmittelstrom aus der Absorptionskolonne zugeführt.

Die Absorption erfolgt in einer Gegenstromabsorptionskolonne, die vorzugsweise mit Dualflowböden oder Ventilböden bestückt ist, und die von oben mit Lösungsmittel beaufschlagt wird. Das gasförmige Reaktionsprodukt und gegebenenfalls verdampftes Lösungsmittel aus dem Quenchapparat werden von unten in die Kolonne eingeleitet und anschließend auf Absorptionstemperatur abgekühlt. Die Abkühlung erfolgt vorteilhafterweise durch Kühlkreise, d.h. erwärmtes Lösungsmittel wird aus der Kolonne abgezogen, in Wärmetauschern abgekühlt und wieder an einer Stelle oberhalb der Abzugsstelle der Kolonne zugeführt. Nach der Absorption befinden sich alle Schwersieder, der größte Teil der Acrylsäure und ein Teil der Leichtsieder im Lösungsmittel.

Das verbleibende, nicht absorbierte Reaktionsgas wird weiter abgekühlt, um den kondensierbaren Teil der leichtsiedenden Nebenkomponenten insbesondere Wasser, Formaldehyd und Essigsäure, durch Kondensation davon abzutrennen. Dieses Kondensat wird im folgenden Sauerwasser genannt. Der verbleibende Gasstrom besteht überwiegend aus Stickstoff, Kohlenoxiden und nicht umgesetzten Edukten. Vorzugsweise wird dieser teilweise wieder als Verdünnungsgas, im folgenden Kreisgas genannt, den Reaktionsstufen zugeführt. Der Lunstickstoff und ein Teil der nichtkondensierten Nebenkomponenten werden als Abgas ausgeschleust und vorzugsweise verbrannt.

Das Absorbat, ein flüssiges Gemisch von Acrylsäure, Leichtsiedern, Mittelsiedern und Schwersiedern in einem hochsiedenden Lösungsmittel, ist Ausgangsgemisch für die Stufe I des erfindungsgemäßen Verfahrens. Das Absorbat kann gegebenenfalls vor der Zuführung in Stufe I zunächst zur Erhöhung des Acrylsäuregehalts aufkonzentriert werden, insbesondere durch partielle Verdampfung.

### Stufe I

Das Ausgangsgemisch enhält bevorzugt 77 bis 90 Gew.-% (Meth)acrylsäure, 9 bis 20 Gew.-% hochsiedendes Lösungsmittel und 1 bis 3 Gew.-% der Summe aus Leichtsiedern, Mittelsiedern und Schwersiedern. Es wird einer destillativ arbeitenden Abtriebskolonne auf dem obersten Kolonnenboden zugeführt. Die Abtriebskolonne kann grundsätzlich jede Art von trennwirksamen Einbauten aufweisen, bevorzugt Dual-Flow- oder Ventilböden, jedoch auch Füllkörper oder strukturierte Packungen. Die Abtriebskolonne weist einen Sumpfverdampfer und gegebenenfalls einen Kondensator am Kolonnenkopf auf.

Der aufzutrennende flüssige Strom läuft abwärts durch die Kolonne und im Gegenzug steigt Dampf, vorwiegend dampfförmige Acrylsäure, aus dem Sumpf nach oben und strippt dabei die Leichtsieder aus der Flüssigkeit, so daß der im Sumpf ankommende Flüssigkeitsstrom (b) nahezu leichtsiederfrei ist. Bei der Strippung bleiben dagegen die Mittelsieder und Schwersieder überwiegend in der Flüssigkeit und reduzieren die Polymerisationsneigung der Acrylsäure während des Strippvorgangs.

Der Brüdenstrom am Kolonnenkopf (Teilstrom a) wird bevorzugt kondensiert und zur Absorptionsstufe oder zum Direktkondensator in das hochsiedende Lösungsmittel rezirkuliert. Möglich ist jedoch auch, den Brüdenstrom in Dampfform, gegebenenfalls nach Verdichtung, der Absorptionsstufe zuzuführen.

Die bevorzugten Betriebsparameter in der Abtriebskolonne sind:
Kopfdruck < 200 mbar, insbesondere < 100 mbar, besonders bevorzugt < 50 mbar,
Sumpftemperatur < 140°C, insbesondere < 120°C, besonders bevorzugt <100°C und
Acrylsäurekonzentration im Sumpf 5 bis 15 Gew.-%, besonders bevorzugt 8 bis 12 Gew.-%.

### Stufe II

Die Gewinnung der Acrylsäure aus dem Teilstrom b erfolgt bevorzugt durch Auftrennen des Teilstroms b in einen ersten, Rohacrylsäure enthaltenden Teilstrom, der gegebenenfalls weiter gereinigt werden kann, sowie einen Teilstrom c. Die Verfahrensstufe II erfolgt destillativ in einer Auftriebskolonne.

Bevorzugt haben die Abtriebskolonne für die Verfahrensstufe I und die Auftriebskolonne für die Verfahrensstufe II einen gemeinsamen Sumpf. Der als Ergebnis der Verfahrensstufe I im gemeinsamen Sumpf von Abtriebs- und Auftriebskolonne anfallende Teilstrom b wird in Verfahrensstufe II in der Auftriebskolonne aufgetrennt. Dabei fällt im Kolonnensumpf ein Teilstrom c an, der vorwiegend das Lösungsmittel enthält und der, gegebenenfalls nach einer Reinigung, insbesondere durch Verdampfung in einem Quench, in die Absorptionsstufe rezirkuliert wird. In der Auftriebskolonne steigt der vollständig oder nahezu vollständig von Leichtsiedern freie Dampf nach oben, wobei die Mittelsieder und Schwersieder durch den flüssigen Rücklauf aus dem Dampf ausgewaschen werden. Am Kolonnenkopf wird der Brüden kondensiert, ein Teil wird am Kopf als Produkt abgezogen, der Rest ist flüssiger Rücklauf. Das Produkt ist Acrylsäure, die weitgehend frei ist von Leichtsiedern, Mittelsiedern und Schwersiedern.

Die Erfindung wird im folgenden anhand einer Zeichnung sowie von Ausführungsbeispielen näher erläutert.

Es zeigen:
- Figur 1:: die schematische Darstellung eines Verfahrens zur kontinuierlichen Gewinnung von Acrylsäure aus einem flüssigen Gemisch mit einem hochsiedenden Lösungsmittel sowie mit Leichtsiedern, Mittelsieder und Schwersiedern nach dem Stand der Technik,
- Figur 2:: die schematische Darstellung eines Verfahrens zur kontinuierlichen Gewinnung von Acrylsäure aus einem flüssigen Gemisch mit einem hochsiedenden Lösungsmittel sowie mit Leichtsiedern, Mittelsiedern und Schwersiedern nach der Erfindung.

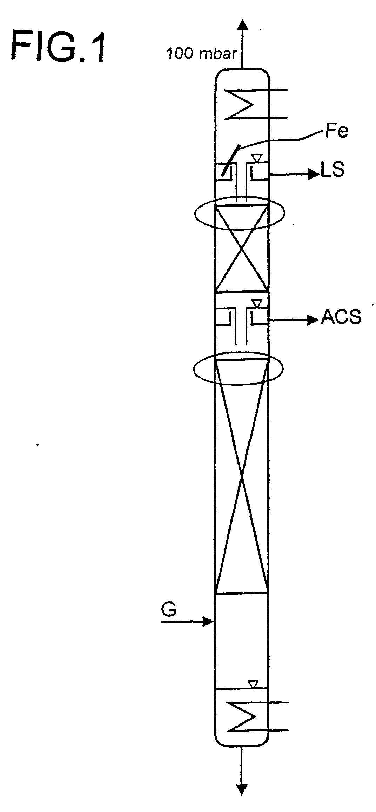

Nach dem herkömmlichen Verfahren (Figur 1) wird das flüssige Gemisch (G), das neben Acrylsäure, Leichtsieder, Mittelsieder und Schwersieder in einem hochsiedenden Lösungmittel enthält, dem unteren Teil einer Destillationkolonne zugeführt. Aus dem Kolonnensumpf wird ein Gemisch abgezogen, das überwiegend das Lösungmittel sowie Schwersieder und Mittelsieder enthält. Der dampfförmige Strom, der überwiegend Acrylsäure sowie die Leichsieder enthält, steigt nach oben, die Leichtsieder (LS) werden am Kolonnenkopf und die Acrylsäure (ACS) wird über einen Seitenabzug gewonnen. Da die Schwersieder und Mittelsieder vollständig oder nahezu vollständig aus dem Sumpf abgezogen werden, enthält der flüssige Rücklauf in der Kolonne nahezu keine Schwersieder und Mittelsieder.

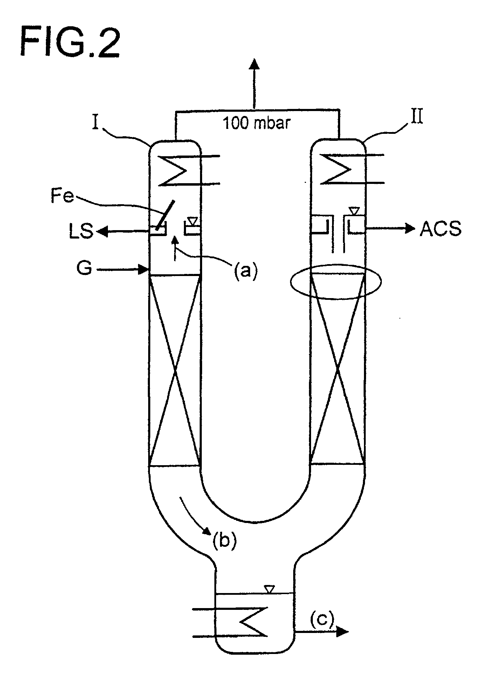

Figur 2 stellt demgegenüber schematisch ein Verfahren nach der Erfindung dar: dasselbe flüssige Ausgangsgemisch, das Acrylsäure sowie Leichtsieder, Mittelsieder und Schwersieder in einem hochsiedenden Lösungsmittel enthält (G) wird auf den Kopf einer Abtriebskolonne I gegeben, die mit einem Sumpfverdampfer und gegebenenfalls einem Kondensator am Kopf ausgestattet ist. Am Kopf der Kolonne I wird ein die Leichtsieder (LS) enthaltender Teilstrom a abgezogen, während der Teilstrom b im Kolonnensumpf anfällt und dieser anschließend in der Auftriebskolonne II in einen Teilstrom c, der aus dem Sumpf abgezogen wird sowie einen Teilstrom, der die Acrylsäure (ACS) enthält, am Kolonnenkopf aufgetrennt wird. Abtriebskolonne I und Auftriebskolonne II sind mit einem gemeinsamen Sumpf ausgestattet

Der Einfluß der Trennfolge auf die Polymerisationsneigung von Acrylsäure wird im folgenden anhand von Beispielen näher erläutert. Zum Vergleich wurde dasselbe flüssige Ausgangsgemisch jeweils einer herkömmlichen Destillationskolonne sowie einer Vorrichtung mit Abtriebskolonne I, Auftriebskolonne II und gemeinsamem Sumpf entsprechend der Erfindung zugeführt. Die Kolonnen hatten jeweils einen Durchmesser von 30 mm. Zur Verkürzung der Versuchsdauer wurde in einigen Beispielen Eisenblech (Fe) eingesetzt, das ein starker Polymerisationsförderer ist.

Das aufzutrennende flüssige Gemisch (G) wies in allen Beispielen die gleiche Zusammensetzung, mit folgenden Hauptkomponenten (jeweils in Gew.-%) auf:

| | |
|---|---|
| 61,92 | Diphyl |
| 17,32 | Acrylsäure |
| 15,50 | Dimethylphthalat |
| 1,72 | Diacrylsäure |
| 0,80 | Benzoesäure |
| 0,21 | Wasser |
| 0,11 | Benzaldehyd |
| 0,04 | Essigsäure |
| 0,006 | 2-Furaldehyd |
| 0,001 | Allyacrylat |

### Beispiel 1

Das aufzutrennende flüssige Gemisch (G) mit der oben angegebenen Zusammensetzung wurde jeweils einer Kolonne nach dem Stand der Technik (Figur 1) sowie eine Vorrichtung mit Abtriebskolonne, Auftriebskolonne und gemeinsamen Sumpf nach der Erfindung (Figur 2) mit Eisenblech (Fe) als Polymerisationsförderer und ohne trennwirksame Einbauten (Füllkörper) zugeführt. Die Trennqualität war schlecht. Bei einer Versuchsdauer von 280 min fand weder am Produktabzug (ACS) noch am Leichtsiederabzug (LS) beim herkömmlichen Verfahren wie auch beim Verfahren gemäß der Erfindung Polymerisation statt.

### Beispiel 2

Die Versuchsbedingungen unterschieden sich von Beispiel 1 dadurch, daß kein Polymerisationsförderer (Eisenblech) verwendet wurde, dagegen trennwirksame Einbauten in Form von Glasringen mit 8 mm Durchmesser. Es wurde eine mittlere Trennqualität erreicht. Bei einer Versuchsdauer von 140 min wurde beim Verfahren nach dem Stand der Technik am Produktabzug keine Polymerisation beobachtet, dagegen am Leichtsiederabzug Polymerisation nach 125 min. Beim erfindungsgemäßen Verfahren wurde dagegen bei einer Versuchsdauer von 140 min weder am Produkt- noch am Leichtsiederabzug Polymerisation beobachtet.

### Beispiel 3

Die Versuchsbedingungen unterschieden sich von den Bedingungen des Beispiels 2 insofern, als Glasringe mit einem kleineren Durchmesser (5 mm) und somit besserer Trennwirkung eingesetzt wurden. Bei einer Versuchsdauer von 120 min wurde am Produktabzug beim Verfahren nach dem Stand der Technik keine Polymerisation beobachtet, dagegen am Leichtsiederabzug, nach 105 min. Beim Verfahren gemäß der Erfindung fand bei einer Versuchsdauer von 120 min keine Polymerisation statt.

### Beispiel 4

Die Versuchsbedingungen entsprachen Beispiel 3, bis auf die Verwendung von Glasringen mit kleinerem Durchmesser von 3 mm, als Füllkörper mit sehr guter Trennqualität. Beim Verfahren nach dem Stand der Technik wurde am Produktabzug nach 48 min und am Leichtsiederabzug nach 70 min Polymerisation beobachtet. Dagegen fand beim Verfahren gemäß der Erfindung während einer Versuchsdauer von 90 min keine Polymerisation statt.

### Beispiel 5

Gegenüber Beispiel 2 wurde das Füllkörpermaterial geändert, und zwar wurden Metallwendel aus V2A-Stahl mit 5 mm eingesetzt, die eine sehr gute Trennqualität bewirkten. Beim Verfahren nach dem Stand der Technik wurde am Produktabzug nach 35 min Polymerisation beobachtet, am Leichtsiederabzug keine Polymerisation bei einer Versuchsdauer von 90 min. Beim erfindungsgemäßen Verfahren setzte dagegen die Polymerisation am Produktabzug erst nach 65 min ein, am Leichtsiederabzug fand bei einer Versuchsdauer von 90 min keine Polymerisation statt.

### Beispiel 6

Die Versuchsbedingungen entsprachen Beispiel 5. Zusätzlich wurde Eisenblech (Fe) als Polymerisationsförderer eingesetzt. Beim Verfahren nach dem Stand der Technik wurde am Produktabzug bereits nach 5 min Polymerisation beobachtet, am Leichtsiederabzug nach 25 min. Dagegen fand nach dem erfindungsgemäßen Verfahren Polymerisation am Produktabzug erst nach 15 min statt und bei einer Versuchsdauer von 45 min keine Polymerisation am Leichtsiederabzug.In der nachfolgenden Tabelle 1 sind die Versuchsergebnisse zusammengefaßt:

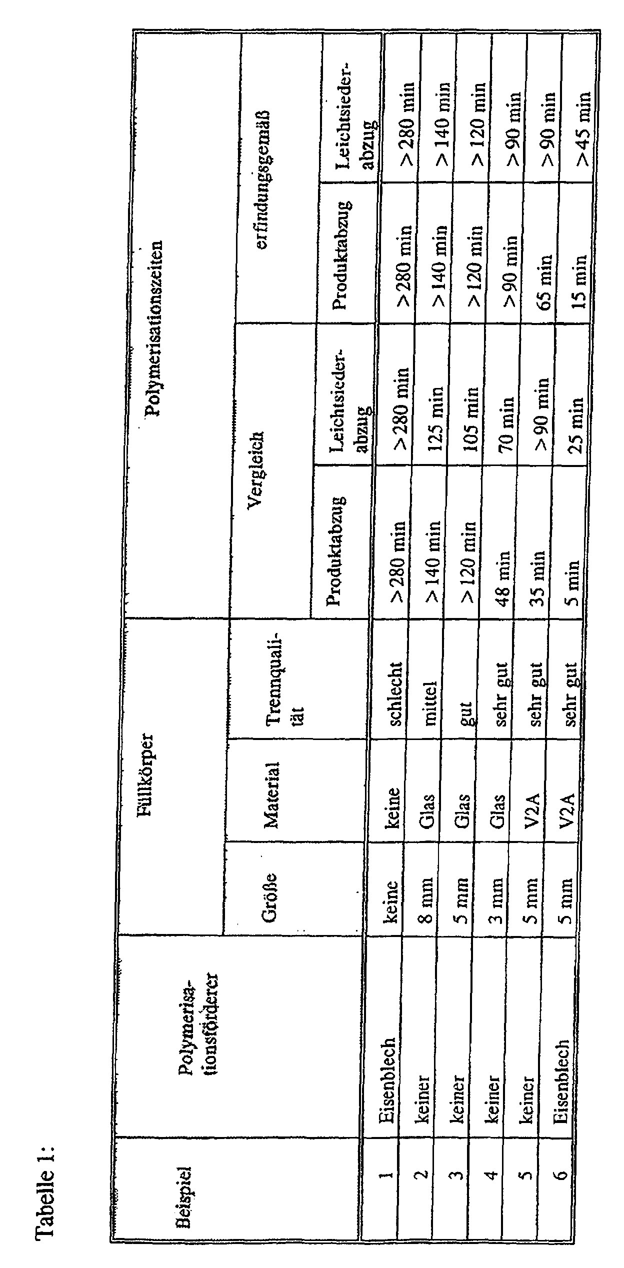

Die Zusammensetzung der Ströme am Leichtsiederabzug (LS) sowie am Produktabzug (ACS), jeweils für das Verfahren nach dem Stand der Technik sowie nach der Erfindung, die insbesondere die jeweils erreichte Trennqualität verdeutlicht, ist in der nachfolgenden Tabelle 2 angegeben:

### Zusammensetzung der Ströme (Angaben in Gew.-%):

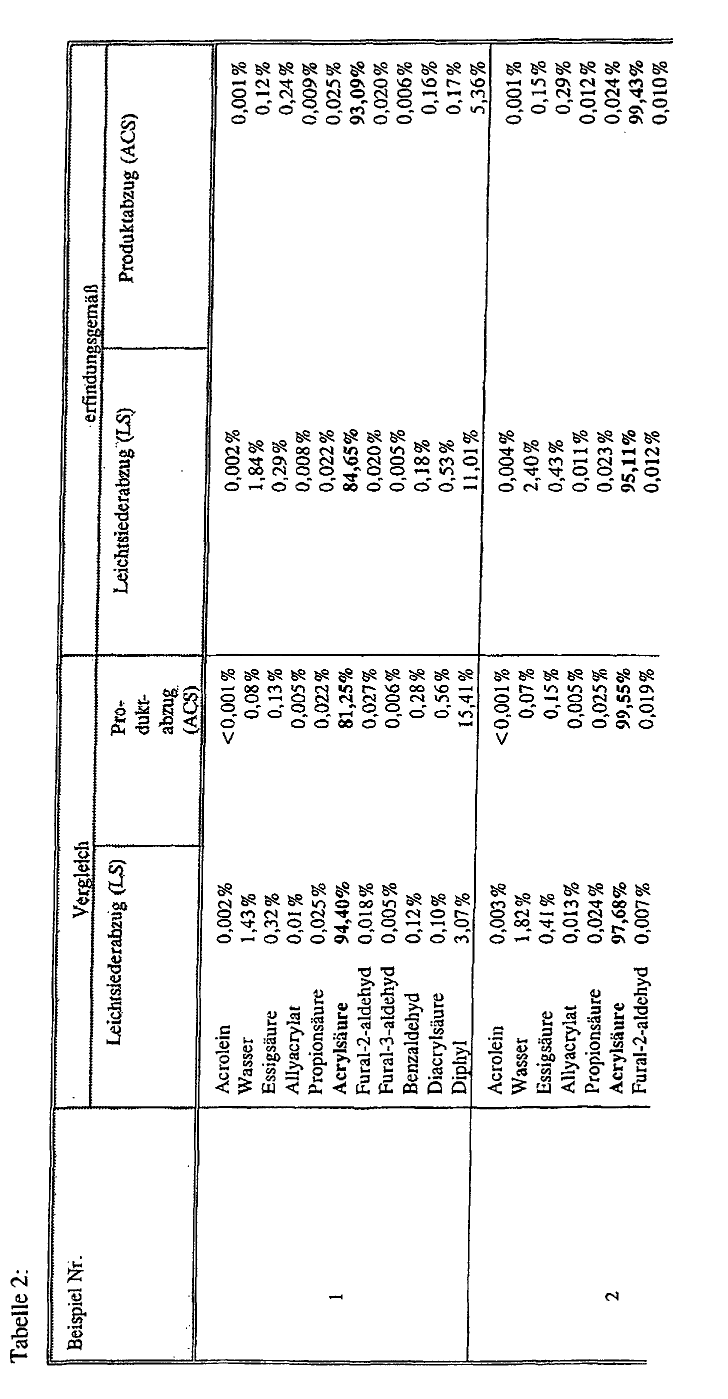

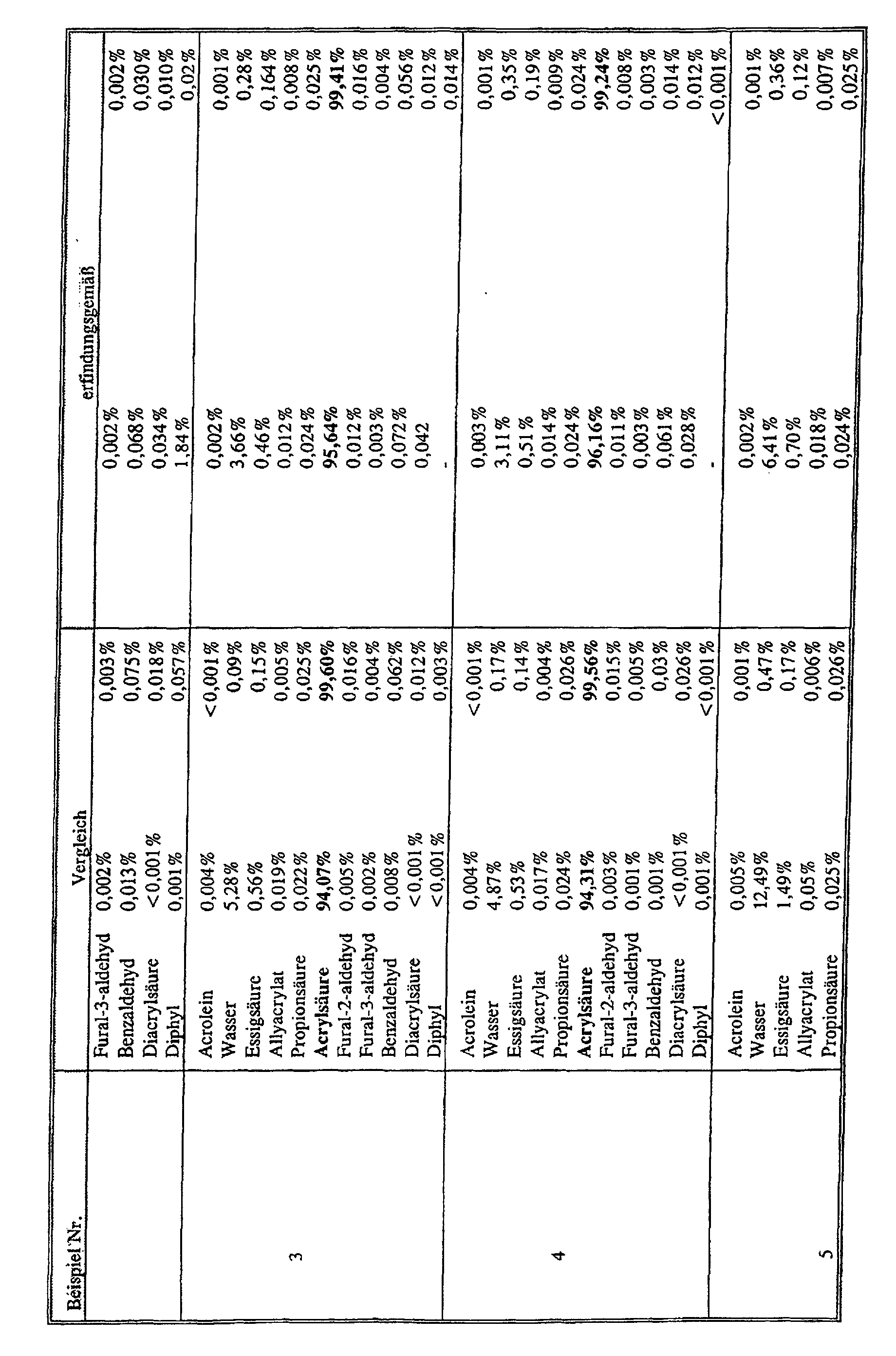

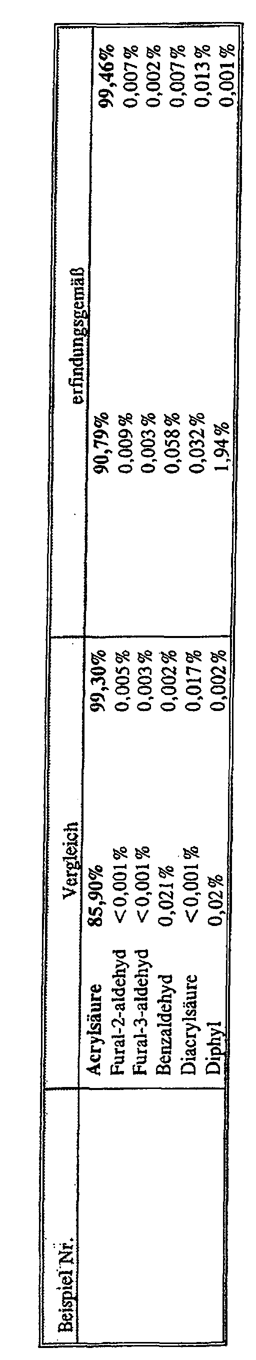

## Patentansprüche

1. Verfahren zur kontinuierlichen Gewinnung von (Meth)acrylsäure aus einem flüssigen Ausgangsgemisch mit einem Gehalt an (Meth)acrylsäure, einem hochsiedenden organischen Lösungsmittel sowie mit Leichtsiedern, Mittelsiedern und Schwersiedern, das heißt Verbindungen, die jeweils durch ihren relativen Siedepunkt gegenüber (Meth)acrylsäure definiert sind, **dadurch gekennzeichnet, daß**
I das Gemisch destillativ in einer Abtriebskolonne in einen ersten Teilstrom (a), der neben (Meth)acrylsäure die Leichtsieder sowie jeweils einen Teil der Mittelsieder und Schwersieder enthält, und einen zweiten Teilstrom (b) aufgetrennt wird, der den überwiegenden Teil der (Meth)acrylsäure enthält und der vollständig oder nahezu vollständig frei von Leichtsiedern ist, indem dampfförmige, aus dem Sumpf der Abtriebskolonne aufsteigende (Meth)acrylsäure die Leichtsieder ohne Zuführung von Inertgas aus der Flüssigkeit strippt,
und daß
II die (Meth)acrylsäure aus dem Teilstrom (b) destillativ in einer Auftriebskolonne gewonnen wird.

2. Verfahren nach Anspruch 1, **dadurch gekennzeichnet, daß** das flüssige Ausgangsgemisch 77 bis 90 Gew.-% (Meth)acrylsäure, 9 bis 20 Gew.-% hochsiedendes Lösungsmittel und 1 bis 3 Gew.-% der Summe aus Leichtsiedern, Mittelsiedern und Schwersiedern, enthält.

3. Verfahren nach Anspruch 1 oder 2, **gekennzeichnet durch** folgende Betriebsparameter in der Abtriebskolonne:
Kopfdruck < 200 mbar, insbesondere < 100 mbar, besonders bevorzugt < 50 mbar,
Sumpftemperatur < 140°C, insbesondere < 120°C, besonders bevorzugt < 100°C und
Acrylsäurekonzentration im Sumpf 5 bis 15 Gew.-%, besonders bevorzugt 8 bis 12 Gew.-%.

4. Verfahren nach einem der Ansprüche 1 bis 3, **dadurch gekennzeichnet, daß** in der Verfahrensstufe II der Teilstrom (b) in einen ersten, Roh-(Meth)acrylsäure enthaltenden Teilstrom, der gegebenenfalls weiter gereinigt werden kann, sowie einen Teilstrom (c) aufgetrennt wird.

5. Verfahren nach einem der Anprüche 1 bis 4, **dadurch gekennzeichnet, daß** die Abtriebskolonne für die Verfahrensstufe I und die Auftriebskolonne für die Verfahrensstufe II einen gemeinsamen Sumpf aufweisen.

## Claims

1. A process for the continuous recovery of (meth)acrylic acid from a liquid starting mixture containing (meth)acrylic acid, a high-boiling organic solvent and low boilers, medium boilers and high boilers, that is compounds which are each defined by their relative boiling point with respect to (meth)acrylic acid, wherein
I the mixture is separated by distillation in a descending stripping column into a first part-stream (a), which, in addition to (meth)acrylic acid, contains the low boilers and a part each of the medium boilers and high boilers, and a second part-stream (b), which contains the predominant part of the (meth)acrylic acid and is completely or virtually completely free of low boilers, in that (meth)acrylic acid rising from the bottom of the descending stripping column in vapor form strips the low boilers from the liquid without feeding in inert gas,
and
II the (meth)acrylic acid is recovered from the part-stream (b) by distillation in an ascending stripping column.

2. A process as claimed in claim 1, wherein the liquid starting mixture contains from 77 to 90% by weight of (meth)acrylic acid, from 9 to 20% by weight of high-boiling solvents and from 1 to 3% by weight of the sum of low boilers, medium boilers and high boilers.

3. A process as claimed in claim 1 or 2, comprising the following operating parameters in the descending stripping column:
Top pressure < 200, in particular < 100, particularly preferably < 50, mbar,
bottom temperature < 140°C, in particular < 120°C, particularly preferably < 100°C, and
acrylic acid concentration in the bottom from 5 to 15, particularly preferably from 8 to 12, % by weight.

4. A process as claimed in any of claims 1 to 3, wherein, in process stage II, the part-stream (b) is separated into a first part-stream, which contains crude (meth)acrylic acid and may, if required, be further purified, and a part-stream (c) .

5. A process as claimed in any of claims 1 to 4, wherein the descending stripping column for process stage I and the ascending stripping column for process stage II have a common bottom.

## Revendications

1. Procédé pour la récupération en continu d'acide (méth)acrylique à partir d'un mélange de départ liquide contenant de l'acide (méth)acrylique, un solvant organique à haut point d'ébullition, ainsi que des fractions à bas point d'ébullition, à moyen point d'ébullition et à haut point d'ébullition, c'est-à-dire des composés qui sont à chaque fois définis par leur point d'ébullition relatif vis-à-vis de l'acide (méth)acrylique, **caractérisé en ce que**
I le mélange est séparé par distillation dans une colonne de stripage, en un premier courant partiel (a) qui, outre l'acide (méth)acrylique, contient les constituants à bas point d'ébullition, ainsi qu'à chaque fois une partie des constituants à moyen point d'ébullition et à haut point d'ébullition, et un deuxième courant partiel (b) qui contient la majeure partie de l'acide (méth)acrylique et qui est totalement ou pratiquement totalement exempt de constituants à bas point d'ébullition, **en ce que** de l'acide (méth)acrylique montant sous forme de vapeur du fond de la colonne de stripage élimine du liquide les constituants à bas point d'ébullition, sans amenée de gaz inerte,
et **en ce que**
II l'acide (méth)acrylique est récupéré du courant partiel (b) par distillation dans une colonne de distillation.

2. Procédé selon la revendication 1, **caractérisé en ce que** le mélange liquide de départ contient de 77 à 90% en poids d'acide (méth)acrylique, de 9 à 20% en poids de solvant à haut point d'ébullition et de 1 à 3% en poids de la somme des constituants à bas point d'ébullition, à moyen point d'ébullition et à haut point d'ébullition.

3. Procédé selon la revendication 1 ou 2, **caractérisé par** les paramètres suivants dans la colonne de stripage:
Pression en tête de colonne < 200 mbar, en particulier < 100 mbar, plus préférentiellement < 50 mbar,
Température de fond de colonne < 140°C, en particulier < 120°C, plus préférentiellement < 100°C, et
Concentration d'acide acrylique dans le fond de colonne de 5 à 15% en poids, plus préférentiellement de 8 à 12% en poids.

4. Procédé selon l'une quelconque des revendications 1 à 3, **caractérisé en ce que**, dans l'étape II du procédé, le courant partiel (b) est séparé en un premier courant partiel contenant de l'acide (méth)acrylique brut, qui peut éventuellement être purifié, et un courant partiel (c).

5. Procédé selon l'une quelconque des revendications 1 à 4, **caractérisé en ce que** la colonne de stripage pour l'étape I du procédé et la colonne de stripage pour l'étape II du procédé présentent un fond de colonne commun.
